(19) Europäisches Patentamt
European Patent Office
Office européen des brevets

(11) Veröffentlichungsnummer : **0 247 485 B1**

# (12) EUROPÄISCHE PATENTSCHRIFT

(45) Veröffentlichungstag der Patentschrift : **17.04.91 Patentblatt 91/16**

(51) Int. Cl.$^5$ : **C07D 305/08,** // C07C53/50, C07D249/08

(21) Anmeldenummer : **87107165.0**

(22) Anmeldetag : **18.05.87**

## (54) Verfahren zur Herstellung von Oxetan-3-carbonsäuren.

(30) Priorität : **30.05.86 DE 3618135**

(43) Veröffentlichungstag der Anmeldung : **02.12.87 Patentblatt 87/49**

(45) Bekanntmachung des Hinweises auf die Patenterteilung : **17.04.91 Patentblatt 91/16**

(84) Benannte Vertragsstaaten : **BE CH DE FR GB IT LI NL**

(56) Entgegenhaltungen :
**EP-A- 0 011 793**
**EP-A- 0 048 974**
**DE-A- 1 907 117**

(56) Entgegenhaltungen :
**PATENT ABSTRACTS OF JAPAN, vol 9, no.184, 30 July 1985, Seite (C-294)(1907)**
**PATENT ABSTRACTS OF JAPAN, vol. 9, no. 71, 30 March 1985, Seite (C-272)(1794)**
**PATENT ABSTRACTS OF JAPAN, vol. 9, no. 233, Seite (C-304)(1956), 19 Sept 1985 .**

(73) Patentinhaber : **BAYER AG**
**W-5090 Leverkusen 1 Bayerwerk (DE)**

(72) Erfinder : **Fiege, Helmut, Dr.**
**Walter-Flex-Strasse 23**
**W-5090 Leverkusen 1 (DE)**
Erfinder : **Jautelat, Manfred, Dr.**
**Müllersbaum 28**
**W-5093 Burscheid 1 (DE)**
Erfinder : **Arlt, Dieter, Prof. Dr.**
**Rybniker Strasse 2**
**W-5000 Köln 80 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Die Erfindung betrifft ein neues Verfahren zur Herstellung von teilweise bekannten Oxetan-3-carbonsäuren, die als Zwischenprodukte zur Synthese von Stoffen mit fungizider bzw. herbizider Wirksamkeit verwendbar sind.

Es ist bereits bekannt, daß sich 3-Alkyloxetan-3-carbonsäuren ausgehend von 3-Alkyl-3-hydroxymethyloxetanen herstellen lassen (vgl. DE-PS 1 907 117). Bei dem bekannten Verfahren werden 3-Alkyl-3-hydroxymethyl-oxetane in flüssiger Phase in Gegenwart von Kupfer/Chrom/Barium enthaltenden Katalysatoren bei Temperaturen zwischen 190 und 270°C dehydriert. Die dabei entstehenden 3-Alkyl-oxetan-3-carbonsäureester, die das als Ausgangsmaterial eingesetzte 3-Alkyl-3-hydroxymethyl-oxetan als Alkohol-Komponente enthalten, werden in einem weiteren Reaktionsschritt zu den 3-Alkyl-oxetan-3-carbonsäuren verseift. Nachteilig an diesem Verfahren ist, daß es sich um eine mehrstufige Synthese handelt und die gewünschten 3-Alkyloxetan-3-carbonsäuren nur in geringer Ausbeute und im Gemisch mit anderen Produkten erhältlich sind.

Es wurde nun gefunden, daß sich Oxetan-3-carbonsäuren der Formel

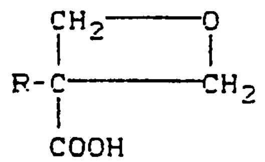

(I)

in welcher

R für Wasserstoff, Alkyl, Cycloalkyl oder gegebenenfalls substituiertes Phenyl steht,

herstellen lassen, indem man 3-Hydroxymethyl-oxetane der Formel

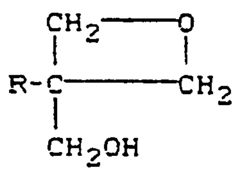

(II)

in welcher

R die oben angegebene Bedeutung hat,

in wäßrig-alkalischem Medium mit Sauerstoff oder Sauerstoff enthaltenden Gasen bei Temperaturen zwischen 0°C und dem Siedepunkt des Reaktionsgemisches an einem Palladium- und/oder Platinkatalysator, gegebenenfalls in Gegenwart eines Aktivators umsetzt und danach gegebenenfalls ansäuert.

Der Verlauf des erfindungsgemäßen Verfahrens ist als äußerst überraschend zu bezeichnen, denn bisher war außer einer thermischen Dehydrierung keine chemische Oxidation von 3-Hydroxymethyl-oxetanen beschrieben worden. Im Hinblick auf den bekannten Stand der Technik konnte also nicht davon ausgegangen werden, daß sich bei der Oxidation von 3-Hydroxymethyl-oxetanen direkt Oxetan-3-carbonsäuren der Formel (I) in einstufiger Reaktion herstellen lassen würden.

Das erfindungsgemäße Verfahren zeichnet sich durch eine Reihe von Vorteilen aus. So sind die benötigten Ausgangsstoffe einfach und auch in größeren Mengen zugänglich. Weiterhin sind auch die erforderlichen Oxidationsmittel und übrigen Reaktionskomponenten preisgünstig und problemlos zu handhaben. Besonders vorteilhaft ist, daß die gewünschten Produkte in sehr hoher Ausbeute und hervorragender Reinheit anfallen, so daß eine weitere destillative Reinigung der thermolabilen, zur Polymerisation neigenden Oxetan-3-carbonsäuren im allgemeinen nicht mehr nötig ist.

Verwendet man 3-Methyl-3-hydroxymethyloxetan als Ausgangsstoff, Sauerstoff als Oxidationsmittel, Palladium und Aktivkohle unter Zusatz von Bismut-nitrat als Katalysator, wäßrige Natronlauge als Reaktionsmedium und verdünnte wäßrige Schwefelsäure zum Ansäuern, so kann der Verlauf des erfindungsgemäßen Verfahrens durch das folgende Formelschema veranschaulicht werden :

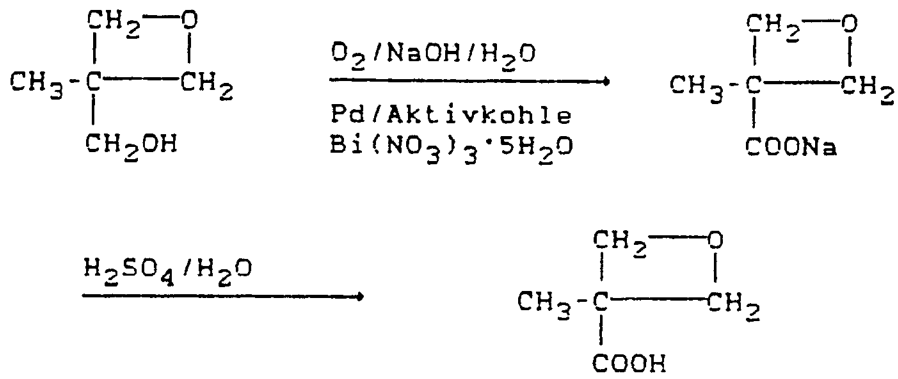


Die bei dem erfindungsgemäßen Verfahren als Ausgangsstoffe benötigten 3-Hydroxymethyl-oxetane sind durch die Formel (II) allgemein definiert. In dieser Formel steht R vorzugsweise für Wasserstoff, Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 6 Kohlenstoffatomen substituiertes Phenyl.

Besonders bevorzugt sind diejenigen Stoffe der Formel (II), in denen R für Wasserstoff, Alkyl mit 1 bis 8 Kohlenstoffatomen, insbesondere mit 1 bis 6 Kohlenstoffatomen, für Cycloalkyl mit 3 bis 6 Kohlenstoffatomen oder für gegenenfalls durch Fluor, Chlor, Brom und/oder Alkyl mit 1 bis 4 Kohlenstoffatomen substituiertes Phenyl steht.

Als Beispiele für 3-Hydroxymethyloxetane der Formel (II) seien genannt :

3-Methyl-3-hydroxymethyl-oxetan, 3-Ethyl-3-hydroxymethyl-oxetan, 3-Propyl-3-hydroxymethyl-oxetan, 3-Isopropyl-3-hydroxymethyl-oxetan und 3-Butyl-3-hydroxymethyl-oxetan, 3-Hydroxymethyl-oxetan, 3-Phenyl-3-hydroxymethyl-oxetan, 3-Cyclohexyl-3-hydroxymethyl-oxetan und 3-(4-chlorphenyl)-3-hydroxymethyl-oxetan.

Die 3-Hydroxymethyl-oxetane der Formel (II) sind bekannt oder lassen sich nach bekannten Methoden in einfacher Weise herstellen (vgl. Houben-Weyl, "Methoden der organischen Chemie", 4. Auflage, Bd. VI/3, Seite 493 ff, Georg-Thieme-Verlag, Stuttgart 1965). So können 3-Hydroxymethyl-oxetane der Formel (II) zum Beispiel durch Abspaltung von Kohlendioxid aus den entsprechenden cyclischen Carbonaten erhalten werden.

Bei der Durchführung des erfindungsgemäßen Verfahrens kommen als Katalysatoren alle üblichen Palladium- und Platin-Katalysatoren sowie deren Gemische in Betracht. Die Katalysatoren können zusätzlich noch mit Aktivatoren oder Gemischen verschiedener Aktivatoren kombiniert werden. Als Aktivatoren kommen hierbei vorzugsweise Blei, Bismut, Bleiverbindungen und Bismutverbindungen sowie deren Gemische in Frage.

Bei der Durchführung des erfindungsgemäßen Verfahrens können das als Katalysator zu verwendende Platin oder Palladium oder Gemische, die diese Metalle enthalten, in üblicher Weise eingesetzt werden. So können die Stoffe in elementarer Form, z.B. als sogenanntes Platin- oder Palladium-Mohr, gegebenenfalls in Kombination mit anderen Platinmetallen, oder auch in Form von Verbindungen, wie z.B. den Oxiden, zugegeben werden.

Das Platin oder das Palladium können auch auf einem Träger aufgebracht sein. Als Träger geeignet sind beispielsweise Aktivkohle, Graphit, Kieselgur, Kieselgel, Spinelle, Aluminiumoxid, Asbest, Calciumcarbonat, Bariumsulfat oder auch organische Trägermaterialien.

Bevorzugt werden als Trägermaterial Aktivkohlen, beispielsweise sogenannte Medizinalkohlen oder aus Holz hergestellte Aktivkohlen, wie sie vielfach für Entfärbungszwecke verwendet werden, eingesetzt.

Der Platin- und/oder Palladium-Gehalt der Trägerkatalysatoren kann innerhalb eines größeren Bereiches variiert werden. Im allgemeinen verwendet man Trägerkatalysatoren, in denen der Gehalt an diesen Metallen zwischen 0,01 und 20 Gew.-%, vorzugsweise zwischen 0,1 und 15 Gew.-%, liegt.

Auch die Mengen, in denen die Platin- und/oder Palladium-Katalysatoren eingesetzt werden, können in einem größeren Bereich variiert werden. Die Mengen hängen unter anderem von der gewünschten Oxidationsgeschwindigkeit ab. Im allgemeinen wählt man die Katalysatormenge so, daß zwischen 0,01 und 20 g, vorzugsweise zwischen 0,05 und 10 g Platin und/oder Palladium pro Mol 3-Hydroxymethyl-oxetan der Formel (II) im Reaktionsgemisch anwesend sind.

Bei der Durchführung des erfindungsgemäßen Verfahrens ist es auch möglich, Platin in Kombination mit Palladium als Katalysator einzusetzen.

Die Aktivität und/oder Selektivität der Platinkatalysatoren wird bei dem erfindungsgemäßen Verfahren

durch die Gegenwart von Blei und/oder Bismut und/oder deren Verbindungen als Aktivator erheblich gesteigert.

Palladiumkatalysatoren weisen auch ohne Zusatz der vorgenannten Aktivatoren schon eine so überraschend hohe Aktivität und Selektivität auf, daß bei ihnen gegebenenfalls auf den Zusatz der vorgenannten Aktivatoren verzichtet werden kann.

Der Zusatz der vorgenannten Aktivatoren wirkt sich auch positiv auf die Wiederverwendbarkeit der Katalysatoren aus.

Die Mengen, in denen die Aktivatoren bei der Durchführung des erfindungsgemäßen Verfahrens gegebenenfalls eingesetzt werden, können in einem größeren Bereich variiert werden. Die Aktivatorwirkung macht sich bereits bei Zusätzen von $5 \times 10^{-6}$ Mol Metall oder Metallverbindung pro Mol 3-Hydroxymethyloxetan bemerkbar. Es können auch 0,1 Mol oder mehr Aktivator pro Mol 3-Hydroxymethyl-oxetan eingesetzt werden, jedoch bieten diese hohen Zusätze im allgemeinen keinen Vorteil. Üblicherweise werden die Aktivatoren in Mengen von etwa $1 \times 10^{-5}$ bis $1 \times 10^{-1}$ Mol, vorzugsweise $2 \times 10^{-5}$ bis $2 \times 10^{-2}$ Mol, pro Mol zu oxidierendem 3-Hydroxymethyl-oxetan zugegeben.

Die bei der Durchführung des erfindungsgemäßen Verfahrens gegebenenfalls als Aktivatoren zu verwendenden Metalle können in elementarer Form und/oder in Form ihrer Verbindungen, z.B. als Oxide, Hydroxide, Oxidhydrate oder Sauerstoffsäuren oder als Salze von Wasserstoffsäuren, wie Chloride, Bromide, Jodide, Sulfide, Selenide, Telluride, oder als Salze von anorganischen Sauerstoffsäuren, wie Nitrate, Nitrite, Phosphite, Phosphate, Arsenite, Arsenate, Antimonite, Antimonate, Bismutate, Stannate, Plumbate, Selenite, Selenate, Tellurite, Tellurate, Borate oder als Salze von Sauerstoffsäuren, die von Übergangsmetallen abstammen, wie Vanadate, Niobate, Tantalate, Chromate, Molybdate, Wolframate, Permanganate, oder als Salze organischer aliphatischer oder aromatischer Säuren, wie Formiate, Acetate, Propionate, Benzoate, Salicylate, Lactate, Mandelate, Glyoxylate, Oxetan-carboxylate, Citrate, Phenolate, oder als Komplexverbindungen oder als metallorganische Verbindung eingesetzt werden.

Die Aktivatoren können im Reaktionsgemisch jeweils löslich, teilweise löslich oder unlöslich sein.

Es ist auch möglich, die Aktivatoren in Kombination mit anderen nicht als Aktivator beanspruchten Elementen oder Verbindungen in das erfindungsgemäße Verfahren einzusetzen.

Die bei der Durchführung des erfindungsgemäßen Verfahrens gegebenenfalls einzusetzenden Aktivatoren können in unterschiedlichen und auch in gemischten Wertigkeitsstufen vorliegen. Es können auch Änderungen in der Wertigkeit während der Reaktion eintreten. Sofern die Aktivatoren nicht bereits als Oxide und/oder Hydroxide zugegeben werden, ist es möglich, daß sie sich im alkalischen Medium ganz oder teilweise in diese umwandeln. Nach der Reaktion kann der Platin und/oder Palladiumkatalysator mit dem Aktivator (soweit dieser ungelöst geblieben ist) abfiltriert und für weitere Oxidationsreaktionen verwendet werden. Verluste an Platin- bzw. Palladiumkatalysator und/oder an Aktivator sind gegebenenfalls zu ersetzen.

Der Aktivator kann als Feststoff, vorzugsweise in feinverteilter Form, oder in gelöster Form den Reaktionskomponenten zugesetzt werden. Man kann den Aktivator auch schon bei der Herstellung des Platin- bzw. Palladium-Katalysators zugeben oder den Platin- bzw. Palladiumkatalysator mit dem Aktivator imprägnieren. Der Aktivator kann auch als Trägermaterial für das Platinmetall dienen.

Die Oxidation nach dem erfindungsgemäßen Verfahren erfolgt in wäßrig-alkalischem Medium bei einem pH-Wert > 7. Die Einstellung des geeigneten pH-Wertes erfolgt durch Zugabe von Alkalien. Als Alkalien kommen Verbindungen der Alkali- und/oder Erdalkalimetalle in Betracht, wie die Hydroxide, Carbonate, Hydrogencarbonate, Phosphate und Borate. Bevorzugt werden die Hydroxide und/oder Carbonate des Natriums und/oder Kaliums als Alkali eingesetzt.

Da bei dem erfindungsgemäßen Verfahren pro Mol gebildeter Säure 1 Mol Alkali ($OH^{\ominus}$) verbraucht wird, liegt die einzusetzende Alkalimenge bei etwa 1 Mol Alkali pro Mol 3-Hydroxymethyl-oxetan. Im allgemeinen werden etwa 1 bis 1,5 Mol Alkali pro Mol 3-Hydroxymethyl-oxetan eingesetzt.

Höhere Verhältnisse können angewendet werden, bringen jedoch gewöhnlich keine wesentlichen Vorteile mehr. Wenn es erwünscht ist, daß vom eingesetzten 3-Hydroxymethyloxetan nur ein Teil zur Oxetan-3-carbonsäure oxidiert wird, kann auch entsprechend weniger Alkali eingesetzt werden.

Das Alkali kann zu Beginn auf einmal oder auch erst im Verlauf der Reaktion intermittierend oder kontinuierlich dem Reaktionsgemisch hinzugefügt werden.

Die 3-Hydroxymethyl-oxetane werden bevorzugt in wäßriger Lösung oxidiert. Es können aber auch noch andere inerte organische Substanzen, beispielsweise Lösungsmittel, wie tert.-Butanol, Aceton, Dioxan und-/oder Toluol anwesend sein. Die 3-Hydroxymethyl-oxetane werden im allgemeinen in Form einer 2 bis 40%igen Lösung eingesetzt. Welche Konzentration zweckmäßig ist, hängt unter anderem von der gewünschten Reaktionsgeschwindigkeit ab. Letztere nimmt bei höheren 3-Hydroxymethyl-oxetan-Konzentrationen allmählich ab. Es ist auch möglich, Gemische verschiedener 3-Hydroxymethyl-oxetane zu oxidieren.

Die Reaktionstemperaturen können bei der Durchführung des erfindungsgemäßen Verfahrens innerhalb eines größeren Bereiches variiert werden. So kann die Reaktionstemperatur zwischen dem Erstarrungspunkt

und dem Siedepunkt des Reaktionsgemisches liegen. Die im Einzelfall anzuwendende Reaktionstemperatur richtet sich unter anderem nach dem Katalysatorsystem, der Katalysatormenge, der Alkalikonzentration, den Substanzeigenschaften der Edukte und Produkte und den technischen Gegebenheiten, wie zum Beispiel gewünschte Reaktionsgeschwindigkeit oder Wärmeabfuhr. Im allgemeinen arbeitet man bei Temperaturen zwischen 0°C und dem Siedepunkt des Reaktionsgemisches, vorzugsweise zwischen 40°C und 100°C.

Die Reihenfolge, in der der Platin- und/oder Palladium-Katalysator sowie gegebenenfalls Aktivator, wäßriges Alkali und 3-Hydroxymethyl-oxetan zusammengegeben werden, ist beliebig. So können der Platin- und/oder Palladium-Katalysator sowie gegebenenfalls Aktivator der Mischung oder Lösung aus wäßrigem Alkali und 3-Hydroxymethyl-oxetan zugesetzt werden. Man kann auch Platin-und/oder Palladium-Katalysator sowie gegebenenfalls Aktivator vorlegen und die Mischung aus wäßrigem Alkali und 3-Hydroxymethyloxetan zusetzen. Schließlich ist es auch möglich, den Platin- und/oder Palladium-Katalysator, einen Teil des wäßrigen Alkalis sowie gegebenenfalls Aktivator vorzulegen und 3-Hydroxymethyl-oxetan dann zusammen mit dem restlichen Alkali hinzuzufügen. Ferner ist es möglich, den Aktivator der Mischung der übrigen Komponenten zuzusetzen.

Im allgemeinen wird das erfindungsgemäße Verfahren so durchgeführt, daß man Sauerstoff oder Sauerstoff enthaltende Gase, wie Luft, mit dem Reaktionsgemisch, das wäßriges Alkali, den Platin- und/oder Palladium-Katalysator, gegebenenfalls Aktivator sowie 3-Hydroxymethyl-oxetan enthält, in intensiven Kontakt bringt. Der Katalysator braucht im Reaktionsgemisch nicht als Pulver suspendiert vorzuliegen, sondern kann auch in gekörnter Form als Festbett angeordnet sein, das von den übrigen Komponenten durchströmt wird.

Der Druck kann bei der Durchführung des erfindungsgemäßen Verfahrens innerhalb eines größeren Bereiches variiert werden. Im allgemeinen arbeitet man unter Drucken zwischen 0,5 und 10 bar. Vorzugsweise führt man die Oxidation bei Atmosphärendruck durch.

Der Verlauf der Umsetzung kann durch Messung der aufgenommenen Sauerstoffmenge verfolgt werden. Die Umsetzung wird abgebrochen, wenn die für die Herstellung der jeweiligen Oxetan-3-carbonsäure theoretisch erforderliche Sauerstoffmenge aufgenommen worden ist. Im allgemeinen hört die Sauerstoffaufnahme in diesem Stadium von selbst auf, oder sie verlangsamt sich deutlich.

Die Aufarbeitung erfolgt bei der Durchführung des erfindungsgemäßen Verfahrens nach üblichen Methoden. Im allgemeinen geht man so vor, daß man den Katalysator sowie gegebenenfalls vorhandenen ungelösten Aktivator zum Beispiel durch Filtration abtrennt. Die erhaltenen Alkalisalzlösungen der Oxetan-3-carbonsäuren können, gegebenenfalls nach vorherigem Eindampfen, als solche weiterverwendet werden. Man kann die Alkalisalzlösungen der Oxetan-3-carbonsäuren auch vollständig, also bis zur Trockene, eindampfen und den verbleibenden Salzrückstand weiterverwenden. Wenn die freien Oxetan-3-carbonsäuren hergestellt werden sollen, geht man im allgemeinen so vor, daß man das verbleibende Reaktionsgemisch, gegebenenfalls nach vorherigem Einengen unter vermindertem Druck, mit verdünnter Mineralsäure ansäuert, dann mit einem in Wasser wenig löslichen organischen Solvens extrahiert und die organische Phase, gegebenenfalls nach vorherigem Trocknen, einengt. Als Mineralsäuren können hierbei vorzugsweise Salzsäure, Schwefelsäure oder Phosphorsäure eingesetzt werden. Als organische Solventien für die Extraktion kommen vorzugsweise Ether, wie Dethylether und Diisopropylether, weiterhin Ketone, wie Methylisobutylketon, und außerdem gegebenenfalls halogenierte aliphatische oder aromatische Kohlenwasserstoffe, wie Methylenchlorid, Chloroform, Tetrachlormethan oder Toluol, in Betracht. Es ist auch möglich, die jeweilige Oxetan-3-carbonsäure aus den zunächst anfallenden wäßrigen Alkalisalz-Lösungen an einem Kationenaustauscher freizusetzen und durch schonendes Eindampfen der wäßrigen Lösung zu isolieren. Bei unvollständigem Umsatz des verwendeten 3-Hydroxymethyl-oxetans kann man dieses vor dem Ansäuern durch Extraktion der wäßrigen Alkalisalz-Lösung mit einem in Wasser wenig löslichen organischen Solvens entfernen, wiedergewinnen und gegebenenfalls erneut als Ausgangsmaterial einsetzen.

Die nach dem erfindungsgmäßen Verfahren herstellbaren Oxetan-3-carbonsäuren sind wertvolle Zwischenprodukte zur Herstellung anderer Stoffe. So können die Oxetan-3-carbonsäuren der Formel (I) zur Synthese von Polyadditionsprodukten eingesetzt werden (vgl. DE-OS 1 907 117).

Außerdem lassen sich die Oxetan-3-carbonsäuren der Formel (I) bzw. deren Salze auch in 2,2-Bis-chlormethyl-al-kancarbonsäure-chloride überführen. So erhält man 2,2-Bischlormethyl-alkancarbonsäurechloride der Formel

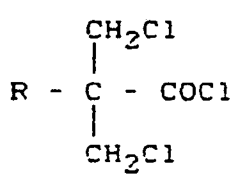

(III)

in welcher
R die oben angegebene Bedeutung hat,
indem man Oxetan-3-carbonsäuren der Formel

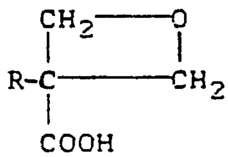

(I)

in welcher
R die oben angegebene Bedeutung hat,
oder deren Salze mit anorganischen Säurechloriden gegebenenfalls in Gegenwärt eines Katalysators und gegebenenfalls in Gegenwart eines Verdünnungsmittels bei Temperaturen zwischen 0°C und dem Siedepunkt des Reaktionsgemisches umsetzt.

Verwendet man 3-Methyl-oxetan-3-carbonsäure als Ausgangsstoff, Thionylchlorid als Säurechlorid und Dimethylformamid (= DMF) als Katalysator, so kann der Verlauf des obigen Verfahrens durch das folgende Formelschema veranschaulicht werden :

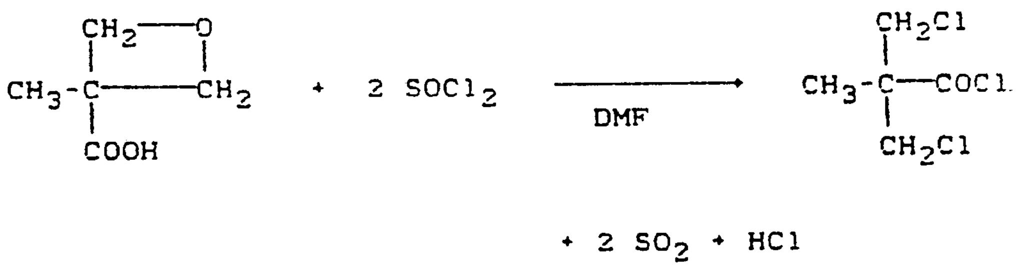


Bei der oben erwähnten Synthese von 2,2-Bischlormethyl-al-kancarbonsäurechloriden der Formel (III) kommen als Ausgangsstoffe die Oxetan-3-carbonsäuren der Formel (I) oder auch deren Salze in Frage. Als Salze können hierbei vorzugsweise Alkalimetall- oder Erdalkalimetall-Salze, wie Natrium-, Kalium-, Magnesium- oder Calcium-Salze, eingesetzt werden. Die Salze der Oxetan-3-carbonsäuren der Formel (I) lassen sich nach üblichen Salzbildungsreaktionen herstellen. Sie fallen bei der Synthese der Oxetan-3-carbonsäuren der Formel (I) nach dem erfindungsgemäßen Verfahren an und können gegebenenfalls nach vorheriger Isolierung für die Weiterreaktion verwendet werden.

Als anorganische Säurechloride kommen bei der Herstellung der Verbindungen der Formel (III) nach dem obigen Verfahren alle üblichen anorganischen Säurechloride in Betracht. Vorzugsweise verwendbar sind Thionylchlorid, Phosphortrichlorid oder Phosphorpentachlorid.

Als Katalysatoren können bei der Herstellung der Verbindungen der Formel (III) nach dem obigen Verfahren alle diejenigen Verbindungen verwendet werden, die bekanntermaßen auch die Umsetzung von Carbonsäuren und ihren Salzen mit anorganischen Säurechloriden zu Carbonsäurechloriden katalysieren (vgl. Houben-Weyl, "Methoden der organischen Chemie", Georg-Thieme-Verlag, Bd. VIII, Seiten 463 ff, Stuttgart 1952, und Bd. E5, Seiten 593 ff (1985)). Vorzugsweise verwendbar sind basische Stickstoffverbindungen, wie tertiäre Amine und Säureamide. Beispielhaft genannt seien Pyridin und Dimethylformamid.

Die Reaktionstemperaturen können bei der Durchführung des obigen Verfahrens zur Herstellung der Verbindungen der Formel (III) innerhalb eines größeren Bereiches variiert werden. Im allgemeinen arbeitet man bei Temperaturen zwischen 20°C und dem Siedepunkt des Reaktionsgemisches.

Als Verdünnungsmittel können bei der Durchführung des obigen Verfahrens zur Herstellung der Verbindungen der Formel (III) alle üblichen inerten organischen Solventien verwendet werden. Vorzugsweise in Betracht kommen aliphatische und aromatische, gegebenenfalls chlorierte Kohlenwasserstoffe sowie Phosphoroxychlorid und Schwefelkohlenstoff.

Bei der Durchführung des obigen Verfahrens zur Herstellung von Verbindungen der Formel (III) geht man im allgemeinen so vor, daß man die Oxetan-3-carbonsäuren bzw. deren Salze mit einer stöchiometrischen Menge oder auch mit einem Überschuß an anorganischen Säurechloriden sowie gegebenenfalls in Gegenwart eines Katalysators umsetzt. Der stöchiometrische Überschuß an anorganischen Säurechloriden kann dabei 5

bis 300%, betragen. Der Katalysator wird im allgemeinen in Mengen von 0,1 bis 20 Gew.-% bezogen auf die eingesetzten Oxetan-3-carbonsäuren bzw. deren Metallsalze zugegeben.

Das Reaktionsgemisch wird bei dem obigen Verfahren nach Maßgabe der Wärme- und/oder Gasentwicklung so lange erhitzt, bis die Umsetzung beendet ist. Zur besseren Reaktionslenkung kann auch in Gegenwart eines inerten Lösungsmittels gearbeitet werden. Im allgemeinen erhitzt man das Reaktionsgemisch bis auf Rückflußtemperatur und behält diese Temperatur bis zur Beendigung der Umsetzung bei. Reaktionsverlauf und Reaktionsende lassen sich nach üblichen Methoden, z.B. durch Gaschromatographie, in einfacher Weise ermitteln.

Die Aufarbeitung des Reaktionsgemisches erfolgt bei dem obigen Verfahren zur Herstellung der Verbindungen der Formel (III) nach üblichen Methoden. Im allgemeinen geht man so vor, daß man das Reaktionsgemisch einer Destillation unterwirft. Nicht umgesetztes anorganisches Säurechlorid wird dabei gesondert aufgefangen und kann zur erneuten Umsetzung verwendet werden. Die Destillationen sind gegebenenfalls im Vakuum und/oder unter Zwischenschaltung einer Kolonne vorzunehmen.

Die 2,2-Bis-chlormethyl-alkancarbonsäurechloride der Formel (III) lassen sich als Ausgangsmaterialien zur Herstellung von herbizid wirksamen Triazinon-Derivaten bzw. zur Synthese von fungizid wirksamen Triazolyl-Derivaten verwenden.

Es kann z.B. das 2,2-Bis-chlorpivaloylchlorid, gegebenenfalls nach vorherigem Austausch der Chloratome gegen Fluoratome, durch Umsetzung mit Trimethylsilylcyanid in die entsprechenden Halogen-pivaloylcyanide überführt werden, welche sich nach bekannten Methoden zu 1,2,4-Triazin-5-on-Derivaten umsetzen lassen (vgl. DE-OS 3 037 300).

Ferner kann z.B. das 2,2-Bis-chlorpivaloylchlorid durch Behandlung mit Kaliumfluorid in das 2,2-Bis-fluorpivaloylfluorid überführt werden, welches mit Malonsäure-monoethylester-Magnesium-Salz zum 2,2-Bis-fluormethylbutan-3-on reagiert. Letzteres läßt sich mit Brom zu dem 2,2-Bisfluormethyl-4-brom-butan-3-on umsetzen, das nach Reaktion mit 1,2,4-Triazol das 2,2-Bis-fluormethyl-4-(1,2,4-triazol-1-yl)-butan-3-on ergibt. Dieses läßt sich durch Umsetzung mit Cyclohexylmethylbromid und Reduktion des zunächst entstehenden Produktes mit Natriumborhydrid in das 2,2-Bis-fluormethyl-5-cyclohexyl-4-(1,2,4-triazol-1-yl)-pentan-3-ol überführen (vgl. DE-OS 3 326 875, DE-OS 2 951 163 und JP-OS 61 572 (1985)). Die genannten Umsetzungen lassen sich durch das folgende Formelschema wiedergeben :

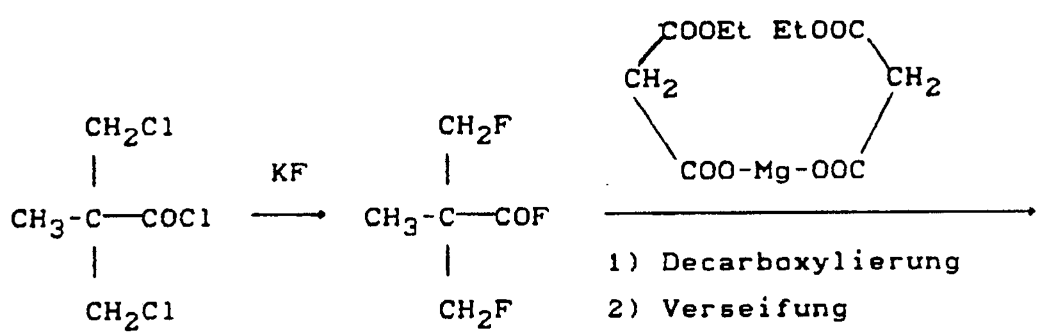

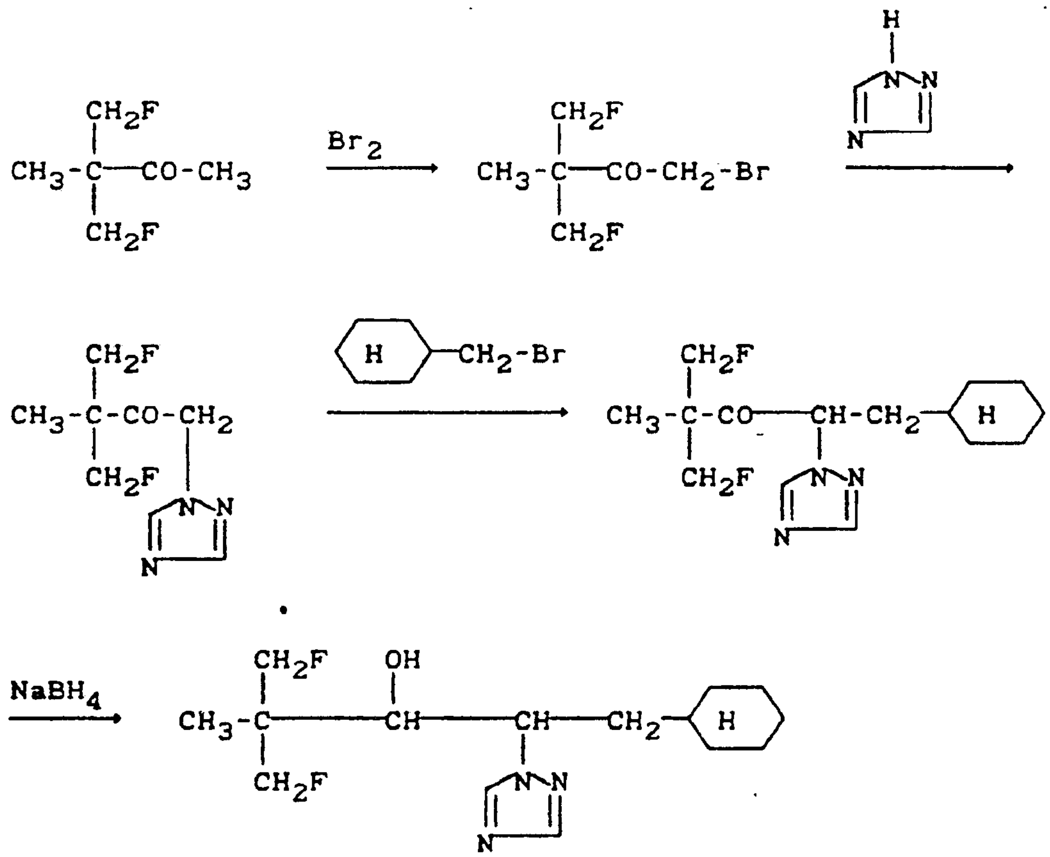


Die Durchführung des erfindungsgemäßen Verfahrens wird durch die folgenden Beispiele veranschaulicht.

Beispiel 1

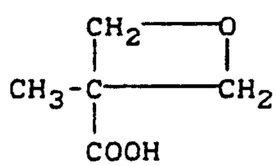

In ein mit Rührer, Innenthermometer und Gaszuleitung versehenes, über einen Heizmantel temperierbares Reaktionsgefäß werden eine Lösung von 20,4 g (0,2 Mol) 3-Methyl-3-hydroxymethyl-oxetan in 100 ml (0,22 Mol) 2,2 m wäßriger Natronlauge, 1 g Aktivkohle mit 5 Gew.-%. Palladiumgehalt und 0,030 g $Bi(NO_3)_3 \cdot 5\ H_2O$ eingebracht.

Nach Verdrängen der Luft aus dem Reaktionsgefäß durch Sauerstoff wird der Rührer eingeschaltet und das Reaktionsgemisch auf 80°C erhitzt. Bei dieser Temperatur wird Sauerstoff unter Normaldruck in die Mischung eingeleitet. Nach 3 Stunden sind 0,2 Mol Sauerstoff aufgenommen und die Reaktion kommt zum Stillstand.

Nach dem Abfiltrieren des Katalysators und Nachwaschen mit 20 ml Wasser wird das Filtrat mit 50%iger Schwefelsäure auf pH 1 angesäuert und mit 2 × 50 ml Methylisobutylketon extrahiert. Nach Abziehen des Methylisobutylketons bei 60°C im Vakuum verbleiben als Rückstand 24 g 3-Methyl-oxetan-3-carbonsäure, die gemäß Gaschromatogramm 3 bis 4% an Methylisobutylketon, aber sonst keine weiteren Verunreinigungen enthält. Die Ausbeute errechnet sich danach zu 99% der Theorie. Fp. 58-60°C (nach Umkristallisation aus Ligroin).

Der abfiltrierte Katalysator kann beim nächsten Ansatz wieder verwendet werden.

Beispiel 2

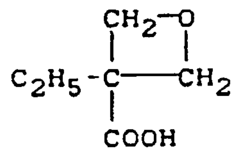

Es wird wie im Beispiel 1 gearbeitet, jedoch mit dem Unterschied, daß 11,6 g (0,1 Mol) 3-Ethyl-3-hydroxymethyl-oxetan in 100 ml 1,2 m wäßriger Natronlauge gelöst und nach Zugabe von 1 g Aktivkohle mit 5 Gew.-% Palladiumgehalt und 30 mg $Bi(NO_3)_3 \cdot 5\ H_2O$ mit Sauerstoff unter Normaldruck bei 80°C oxidiert werden. Nach 90 Minuten sind 0,1 Mol Sauerstoff aufgenommen und die Reaktion kommt zum Stillstand.

Nach dem Abfiltrieren des Katalysators ergibt die Extraktion des mit 50%iger Schwefelsäure auf pH 1 eingestellten Filtrates mit Diethylether 12,9 g eines Produktes, das gemäß Gaschromatogramm zu 99,7% aus 3-Ethyloxetan-3-carbonsäure besteht. Die Ausbeute errechnet sich danach zu 98,9% der Theorie.

Fp. 25°C.

Beispiel 3

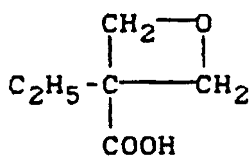

Man arbeitet nach der im Beispiel 2 angegebenen Methode, jedoch ohne Zusatz von $Bi(NO_3)_3 \cdot 5\ H_2O$. Nach 90 Minuten sind 0,1 Mol Sauerstoff aufgenommen. Man erhält 12,7 g eines Produktes, das gemäß Gaschromatogramm zu 99,3% aus 3-Ethyl-oxetan-3-carbonsäure besteht. Die Ausbeute errechnet sich danach zu 97% der Theorie.

Beispiel 4

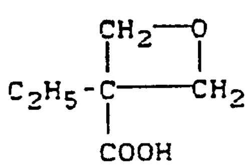

Es wird wie im Beispiel 1 gearbeitet, jedoch mit dem Unterschied, daß 34,8 g (0,3 Mol) 3-Ethyl-3-hydroxymethyloxetan in 150 ml 2,2 m wäßriger Natronlauge gelöst und nach Zugabe von 1,5 g Aktivkohle mit 5 Gew.-% Palladiumgehalt bei 80°C mit Sauerstoff unter atmosphärischem Druck bis zum Stillstand der Sauerstoff-Aufnahme oxidiert werden. Danach stellt man den Rührer ab, läßt den Katalysator absitzen und saugt die überstehende alkalische Lösung über ein Rohr, das am Ende mit einer Fritte versehen ist, bis auf einen kleinen Rest vom Katalysator ab.

Die abgesaugte wäßrig-alkalische Lösung wird zur Entfernung von nicht umgesetztem 3-Ethyl-3-hydroxymethyl-oxetan mit Methylenchlorid extrahiert, dann mit 50%iger Schwefelsäure auf pH 1 angesäuert und zur Gewinnung der 3-Ethyl-oxetan-3-carbonsäure erneut mit Methylenchlorid extrahiert.

Zu dem im Reaktionsgefäß verbliebenen Katalysator werden anschließend wieder 34,8 g (0,3 Mol) 3-Ethyl-3-hydroxymethyl-oxetan und 150 ml 2,2 m wäßrige Natronlauge gegeben. Dann wird wieder wie vorstehend bei 80°C oxidiert und aufgearbeitet.

Anschließend wird das gesamte Verfahren erneut wiederholt. Insgesamt wird der Katalysator auf diese Weise viermal eingesetzt. Danach wird der Versuch abgebrochen.

Die Zeiten bis zum Stillstand der Sauerstoffaufnahme betragen bei der 1., 2., 3, und 4. Verwendung des Katalysators 180, 210, 300 bzw. 510 Minuten.

Isoliert werden insgesamt 146,3 g eines Produktes, das gemäß Gaschromatogramm zu 99,5% aus 3-Ethyl-oxetan-3-carbonsäure besteht. Daraus errechnet sich eine Gesamtausbeute von 93,3% der Theorie.

Beispiel 5

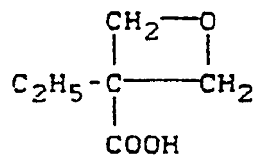

Es wird wie im Beispiel 4 gearbeitet, jedoch mit dem Unterschied, daß bei der 1. Katalysatorverwendung 50 mg $Bi(NO_3)_3 \cdot 5H_2O$ und bei jeder weiteren Katalysatorverwendung 25 mg $Bi(NO_3)_3 \cdot 5H_2O$ als Aktivator zugesetst werden.

Insgesamt wird der Katalysator auf diese Weise 18 x verwendet. Dann wird der Versuch abgebrochen.

Die Zeiten bis zum Stillstand der Sauerstoff-Aufnahme betragen bei der

| | | | |
|---|---|---|---|
| 1. | Katalysatorverwendung | 180 | Minuten |
| 4. | " | 210 | " |
| 8. | " | 270 | " |
| 12. | " | 300 | " |
| 18. | " | 420 | " |

Bei einem Einsatz von insgesamt 18 × 34,8 = 624,4 g 3-Ethyl-3-hydroxymethyl-oxetan werden insgesamt 696,1 g 98,5%ige 3-Ethyl-oxetan-3-carbonsäure gewonnen. Dies entspricht einer Ausbeute von 97,7% d. Theorie.

Der Vergleich mit Beispiel 4 zeigt die positive Wirkung des Aktivators auf die Erhaltung der Aktivität des Katalysators.

Beispiel 6

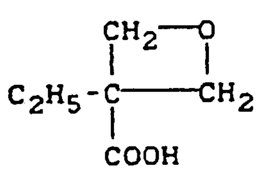

Es wird wie im Beispiel 2 gearbeitet, jedoch bei 50°C. Nach 6 Stunden sind 0,097 Mol Sauerstoff aufgenommen, und die Sauerstoffaufnahme ist noch nicht ganz zum Stillstand gekommen. Man erhält 12,5 g eines Produktes, das gemäß Gaschromatogramm zu 97,8 % aus 3-Ethyl-oxetan-3-carbonsäure besteht. Die Ausbeute errechnet sich danach zu 94% der Theorie.

Beispiel 7

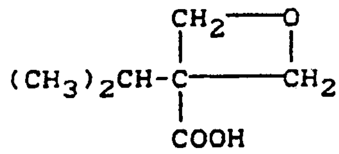

Es wird wie im Beispiel 1 gearbeitet, jedoch mit dem Unterschied, daß 13 g (0,1 Mol) 3-Isopropyl-3-hydroxymethyl-oxetan in 100 ml 1,2 m wäßriger Natronlauge in Gegenwart von 1 g Aktivkohle mit 5 Gew.-% Palladiumgehalt und 30 mg $Bi(NO_3)_3 \cdot 5\ H_2O$ oxidiert herden. Nach 120 Minuten sind 0,1 Mol Sauerstoff aufgenommen, und die Sauerstoffaufnahme ist zum Stillstand gekommen.

Nach dem Abfiltrieren des Katalysators, Ansäuern des Filtrates auf pH 1 und Extraktion mit Diethylether verbleiben nach dem Einengen der organischen Phase unter vermindertem Druck 14,1 g eines Produktes, das zu 94,5% aus 3-Isopropyl-oxetan-3-carbonsäure und zu 3,3% aus 3-Isopropyl-3-hydroxymethyl-oxetan besteht. Die Ausbeute (Selektivität), bezogen auf umgesetztes Ausgangsmaterial errechnet sich danach zu 96% der Theorie.

Fp. 52-54°C.

Bespiele 8 bis 10

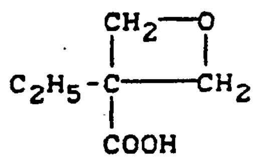

Es wird wie im Beispiel 1 gearbeitet, jedoch mit dem Unterschied, daß 11,6 g (0,1 Mol) 3-Ethyl-3-hydroxymethyl-oxetan in 100 ml 1,2 n wäßriger Natronlauge gelöst und nach Zugabe von 1 g Aktivkohle mit 1 Gew.-% Platingehalt in Abwesenheit (Vergleichsbeispiel) oder Anwesenheit von 30 mg $Bi(NO_3)_3 \cdot 5\ H_2O$ (Beispiel 9) oder 0,5 ml 1 m $Pb(NO_3)_2$-Lösung (Beispiel 10) als Aktivator bei 80°C mit Sauerstoff unter Normaldruck oxidiert werden.

Die Aufarbeitung erfolgt nach Absaugen des Katalysators durch Ansäuern auf pH 1 und Extraktion mit Diethylether. Es werden die in der folgenden Tabelle 1 aufgeführten Ergebnisse erzielt.

Tabelle 1

Einfluß von Blei und Bismut auf die Oxidation von 3-Ethyl-3-hydroxymethyl-oxetan bei 80°C

| Beispiel | Aktivator | $O_2$-Aufnahme | | Ausbeute in % der Theorie |
|---|---|---|---|---|
| | | $\frac{\text{Mol } O_2}{\text{Mol Oxetan}}$ | erforderl. Zeit Stdn. | |
| 8+) | ohne | 0,05 | 7 | < 5 % |
| 9 | $Bi(NO_3)_3$ | 1,00 | 1,5 | 97,3 |
| 10 | $Pb(NO_3)_3$ | 1,00 | 1,0 | 98,5 |

+) zum Vergleich

Beispiel 1

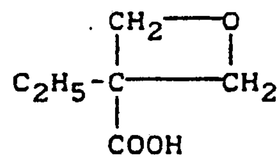

Es wird wie im Beispiel 10 gearbeitet, jedoch bei einer Oxidationstemperatur von 50°C. Nach 2 Stunden sind 0,9 Mol Sauerstoff pro Mol 3-Ethyl-3-hydroxymethyl-oxetan aufgenommen. Nach der Aufarbeitung erhält man 3-Ethyl-oxetan-3-carbonsäure in einer Ausbeute von 86,2% der Theorie.

Beispiel 12

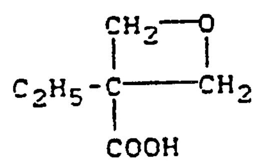

Es wird wie im Beispiel 10 gearbeitet, jedoch bei einer Oxidationstemperatur von 40°C. Nach 2 Stunden sind 0,8 Mol Sauerstoff pro Mol 3-Ethyl-3-hydroxymethyl-oxetan aufgenommen. Nach der Aufarbeitung erhält man 3-Ethyl-oxetan-3-carbonsäure in einer Ausbeute von 86,2% der Theorie.

Herstellung von 2,2-Bis-chlormethyl-alkancarbonsäure-chloriden ausgehend von Oxetan-3-carbonsäuren :

Beispiel 13

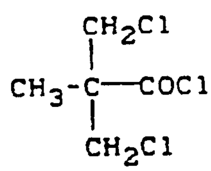

Ein Gemisch aus 11,6 g (0,1 Mol) 3-Methyl-oxetan-3-carbonsäure, 35,7 g (0,3 Mol) Thionylchlorid und 0,2 ml Dimethylformamid wird langsam auf Rückflußtemperatur (etwa 120°C) erhitzt, wobei sich während des Erhitzens Gas entwickelt. Danach wird 5 Stunden bei dieser Temperatur weitergerührt. Die gaschromatographische Untersuchung einer Probe zeigt an, daß sich die eingesetzte 3-Methyl-oxetan-carbonsäure vollständig umgesetzt hat. Anschließend wird überschüssiges Thionylchlorid über eine Destillationsbrüche abdestilliert. Aus dem verbleibenden Rückstand wird bei einem Druck von 10 mbar bei 70-90°C die Hauptfraktion abdestilliert. Man erhält 15,5 g eines Produktes, das gemäß Gaschromatogramm zu 96,1% aus 2,2-Bis-chlormethyl-propancarbonsäurechlorid besteht.

Beispiel 14

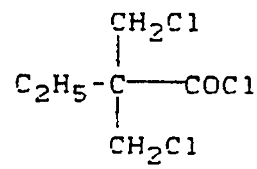

Zu einer Mischung aus 130 g (1 Mol) 3-Ethyl-oxetan-3-carbonsäure und 1 ml Dimethylformamid werden 476 g (4 Mol) Thionylchlorid getropft. Danach wird so auf Rüchflußtemperatur erhitzt, daß ein stetiger Abgasstrom entsteht. Anschließend wird bei Rückflußtemperatur weitergerührt. Nach insgesamt 32 Stunden wird über eine Brücke destilliert. Im Siedebereich von 95 bis 120°C erhält man bei einem Druck von 10 mbar 166,6 g (0,82 Mol) 2,2-Bis-chlormethyl-butancarbonsäurechlorid, das beim Stehen bei Raumtemperatur erstarrt.

Fp. 38-40°C

## Ansprüche

1. Verfahren zur Herstellung von Oxetan-3-carbonsäuren der Formel

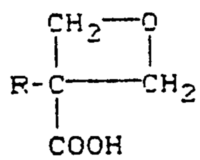

(I)

in welcher
R für Wasserstoff, Alkyl, Cycloalkyl oder gegebenenfalls substituiertes Phenyl steht, dadurch gekennzeichnet, daß man 3-Hydroxymethyl-oxetane der Formel

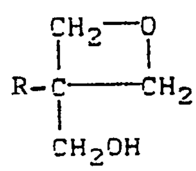

(II)

in welcher
R die oben angegebene Bedeutung hat,
in wäßrig-alkalischem Medium mit Sauerstoff oder Sauerstoff enthaltenden Gasen bei Temperaturen zwischen 0°C und dem Siedepunkt des Reaktionsgemisches an einem Palladium- und/oder Platinkatalysator, gegebenenfalls in Gegenwart eines Aktivators umsetzt und danach gegebenenfalls ansäuert.

2. Verfahren gemäß Anspruch 1, dadurch gekennzeichnet, daß man 3-Hydroxymethyl-oxetane der Formel (II) einsetzt, in denen R für Wasserstoff, Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 6 Kohlenstoffatomen substituiertes Phenyl steht.

3. Verfahren gemäß Anspruch 1, dadurch gekennzeichnet, daß man Platin- und/oder Palladium-Katalysatoren in Form von Trägerkatalysatoren einsetzt.

4. Verfahren gemäß Anspruch 3, dadurch gekennzeichnet, daß man Aktivkohle als Katalysatorträger verwendet.

5. Verfahren gemäß Anspruch 1, dadurch gekennzeichnet, daß man die Umsetzung in Gegenwart eines Aktivators durchführt.

6. Verfahren gemäß Anspruch 5, dadurch gekennzeichnet, daß man Blei, Bismut, Bleiverbindungen, Bismutverbindungen und/oder deren Gemische als Aktivatoren einsetzt.

7. Verfahren gemäß Anspruch 1, dadurch gekennzeichnet, daß man die Umsetzung in Gegenwart wäßriger Lösungen von Alkalimetall- und/oder Erdalkalimetall-Verbindungen durchführt.

8. Verfahren gemäß Anspruch 1, dadurch gekennzeichnet, daß man als Sauerstoff enthaltendes Gas Luft einsetzt.

9. Verfahren gemäß Anspruch 1, dadurch gekennzeichnet, daß man bei Temperaturen zwischen 40°C und 100°C arbeitet.

## Claims

1. Process for the preparation of oxetane-3-carboxylic acids of the formula

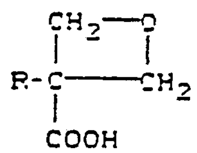

(I)

in which

R represents hydrogen, alkyl, cycloalkyl or optionally substituted phenyl,

characterized in that 3-hydroxymethyl-oxetanes of the formula

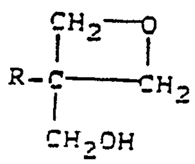

(II)

in which

R has the abovementioned meaning,

are reacted with oxygen or oxygen-containing gases in aqueous alkaline medium at temperatures between 0°C and the boiling point of the reaction mixture on a palladium and/or platinum catalyst, if appropriate in the presence of an activator, and are subsequently acidified, if appropriate.

2. Process according to Claim 1, characterized in that 3-hydroxymethyl-oxetanes of the formula (II) in which R represents hydrogen, alkyl having 1 to 12 carbon atoms, cycloalkyl having 3 to 8 carbon atoms, or phenyl which is optionally substituted by halogen and/or alkyl having 1 to 6 carbon atoms, are employed.

3. Process according to Claim 1, characterized in that platinum and/or palladium catalysts are employed in the form of supported catalysts.

4. Process according to Claim 3, characterized in that activated charcoal is used as catalyst support.

5. Process according to Claim 1, characterized in that the reaction is carried out in the presence of an activator.

6. Process according to Claim 5, characterized in that lead, bismuth, lead compounds, bismuth compounds and/or mixtures thereof are employed as activators.

7. Process according to Claim 1, characterized in that the reaction is carried out in the presence of aqueous solutions of alkali metal and/or alkaline earth metal compounds.

8. Process according to Claim 1, characterized in that air is used as ogen-containing gas.

9. Process according to Claim 1, characterized in that the process is carried out at temperatures between 40°C and 100°C.

## Revendications

1. Procédé de préparation d'acides oxétanne-3-carboxyliques de formule

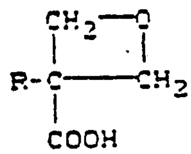

(I)

dans laquelle
R représente un atome d'hydrogène, un groupe alkyle, un groupe cycloalkyle ou un groupe phényle éventuellement substitué,
**caractérisé en ce qu'**on fait réagir des 3-hydroxyméthyl-oxétannes de formule

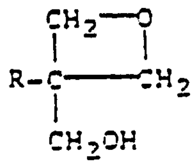

(II)

dans laquelle
R a la signification indiquée ci-dessus,
dans un milieu alcalin aqueux, avec de l'oxygène ou avec des gaz contenant de l'oxygène, à des températures allant de 0°C au point d'ébullition du mélange réactionnel, sur un catalyseur de palladium et/ou de platine, éventuellement en présence d'un activateur, et en ce qu'ensuite, éventuellement, on les acidifie.

2. Procédé selon la revendication 1, **caractérisé en ce qu'**on met en oeuvre des 3-hydroxyméthyl-oxétannes de formule (II) dans laquelle R représente un atome d'hydrogène, un groupe alkyle contenant 1 à 12 atomes de carbone, un groupe cycloalkyle contenant 3 à 8 atomes de carbone ou un groupe phényle éventuellement substitué par un atome d'halogène et/ou par un groupe alkyle contenant 1 à 6 atomes de carbone.

3. Procédé selon la revendication 1, **caractérisé en ce qu'**on met en oeuvre des catalyseurs de platine et/ou de palladium sous forme de catalyseurs à supports.

4. Procédé selon la revendication 3, **caractérisé en ce que**, comme supports de catalyseurs, on utilise des charbons actifs.

5. Procédé selon la revendication 1, **caractérisé en ce qu'**on effectue la réaction en présence d'un activateur.

6. Procédé selon la revendication 5, **caractérisé en ce que**, comme activateurs, on met en oeuvre le plomb, le bismuth, des composés de plomb, des composés de bismuth et/ou des mélanges de ces derniers.

7. Procédé selon la revendication 1, **caractérisé en ce qu'**on effectue la réaction en présence de solutions aqueuses de composés de métaux alcalins et/ou de métaux alcalino-terreux.

8. Procédé selon la revendication 1, **caractérisé en ce que**, comme gaz contenant de l'oxygène, on met en oeuvre de l'air.

9. Procédé selon la revendication 1, **caractérisé en ce l'**on travaille à des températures allant de 40°C à 100°C.